# EUROPÄISCHE PATENTANMELDUNG

(11) **EP 4 509 056 A1**
(43) Veröffentlichungstag der Anmeldung: **19.02.2025**
(21) Anmeldenummer: 23191506.7
(22) Anmeldetag: 15.08.2023
(51) Int. Cl.: A61B 6/04

(54) **MEDIZINTECHNISCHE BILDERFASSUNG MIT VERBESSERTER REGISTRIERUNG**

(71) Anmelder: Siemens Healthineers AG, 91301 Forchheim (DE)
(72) Erfinder: Dickmann, Christoph, 90403 Nürnberg (DE); Requardt, Martin, 90425 Nürnberg (DE)
(74) Vertreter: Siemens Healthineers Patent Attorneys

(57) **Zusammenfassung**

Eine bildgebende medizintechnische Anlage weist ein Erfassungssystem (1) auf, mittels dessen Daten (D) eines in einem Erfassungsbereich (3) des Erfassungssystems (1) befindlichen Objekts (2) erfassbar sind, aus denen entsprechende Bilder (B) des Objekts (2) generierbar sind. Die bildgebende medizintechnische Anlage weist weiterhin einen Liegentisch (5) auf, der einen Unterbau (6) und ein Liegenbrett (7) aufweist. Das Liegenbrett (7) ist auf dem Unterbau (6) zumindest in einer Längsrichtung (x) über einen Verfahrbereich verfahrbar, so dass sich je nach Positionierung des Liegenbretts (7) in der Längsrichtung (x) unterschiedliche Bereiche des Liegenbretts (7) in dem Erfassungsbereich (3) befinden. Auf dem Liegenbrett (7) ist eine Patientenauflage (9) angeordnet, die zusammen mit dem Liegenbrett (7) zumindest in der Längsrichtung (x) verfahren wird. In der Patientenauflage (9) sind in der Längsrichtung (x) im Wesentlichen über den Verfahrbereich verteilt an ersten Markierungsorten erste Markierungen (10) angeordnet. Die ersten Markierungen (10) sind, sofern sich die jeweilige erste Markierung (10) im Erfassungsbereich (3) befindet, in den generierten Bildern (B) erkennbar. Die ersten Markierungsorte und die ersten Markierungen (10) sind derart aufeinander abgestimmt, dass aus den in den generierten Bildern (B) erkennbaren ersten Markierungen (10) der jeweilige erste Markierungsort ableitbar ist.

## Beschreibung

Die vorliegende Erfindung geht aus von einer bildgebenden medizintechnischen Anlage,
- wobei die bildgebende medizintechnische Anlage ein Erfassungssystem aufweist, mittels dessen Daten eines in einem Erfassungsbereich des Erfassungssystems befindlichen Objekts erfassbar sind, aus denen entsprechende Bilder des Objekts generierbar sind,
- wobei die bildgebende medizintechnische Anlage einen Liegentisch aufweist,
- wobei der Liegentisch einen Unterbau und ein Liegenbrett aufweist,
- wobei das Liegenbrett auf dem Unterbau zumindest in einer Längsrichtung über einen Verfahrbereich verfahrbar ist, so dass sich je nach Positionierung des Liegenbretts in der Längsrichtung unterschiedliche Bereiche des Liegenbretts in dem Erfassungsbereich befinden,
- wobei auf dem Liegenbrett eine Patientenauflage angeordnet ist, die zusammen mit dem Liegenbrett zumindest in der Längsrichtung verfahren wird.

Unabhängig vom grammatikalischen Geschlecht eines bestimmten Begriffs (hier des Wortes "Patient") sind Personen mit männlicher, weiblicher und anderer Geschlechteridentität mit umfasst.

Derartige bildgebende medizintechnische Anlagen sind allgemein bekannt. Insbesondere Magnetresonanzanlagen und Computertomographen sind derartige Anlagen.

Bildgebende medizintechnische Anlagen werden einerseits für rein diagnostische Zwecke und andererseits bei der Planung von therapeutischen Maßnahmen eingesetzt, insbesondere auch bei der Planung von strahlentherapeutischen Maßnahmen.

Bei strahlentherapeutischen Maßnahmen ist es von überragender Bedeutung, den Patienten exakt zu positionieren, so dass die therapeutische Strahlung an genau dem geplanten Ort des Patienten wirkt. Der geplante Ort wird in der Regel anhand einer vorherigen diagnostischen Bilderfassung festgelegt. Die Positionierung im Rahmen der strahlentherapeutischen Maßnahme ist umso genauer möglich, je genauer und reproduzierbarer der Patient bei der vorhergehenden, der Planung zugrunde liegenden diagnostischen Bilderfassung positioniert war.

Die exakte und reproduzierbare Positionierung des Patienten im Rahmen der strahlentherapeutischen Maßnahme als solcher ist meist ohne weiteres möglich. Die Problematik besteht darin, die Positionierung des Patienten bei der vorhergehenden, der Planung zugrunde liegenden diagnostischen Bilderfassung so vorzunehmen, dass diese mit der Positionierung des Patienten im Rahmen der späteren strahlentherapeutischen Maßnahme übereinstimmt.

Es ist denkbar, die Registrierung des mittels der bildgebenden medizintechnischen Anlage erfassten bzw. generierten Bildes anhand von signifikanten Strukturen ("landmarks") vorzunehmen, die in dem generierten Bild des Patienten enthalten sind. Dies ist zum einen jedoch nicht stets zuverlässig möglich und weist zum anderen relativ große Ungenauigkeiten auf. Weiterhin können die Liegentische von bildgebenden medizintechnischen Anlagen zwar eine vorgegebene Position exakt anfahren. Über die Lebensdauer des Systems gesehen muss diese Positioniergenauigkeit jedoch auch erhalten bleiben. Daher ist immer wieder eine erneute Kalibrierung erforderlich. Anderenfalls können die Toleranzen für strahlentherapeutische Anwendungen zu groß werden.

Patientenauflagen für strahlentherapeutische Maßnahmen weisen zwar in der Regel seitlich ein Indexsystem auf. Dieses Indexsystem (im Ergebnis ein von einem Menschen ablesbarer Maßstab) ist jedoch in dem Bild, welches anhand der mittels der bildgebenden medizintechnischen Anlage erfassten Daten generiert wird, nicht mehr erkennbar.

Eine bildbasierte Prüfung der Genauigkeit bei der Positionierung eines Patienten auf der Patientenauflage ist daher im Stand der Technik nicht möglich. Es müssen vielmehr zusätzliche und externe Messhilfen verwendet werden.

Im Stand der Technik ist bekannt, die Positionierung der Patientenauflage durch die Verwendung von Phantomen zu ermitteln. Derartige Phantome können an vorbestimmten Stellen der Patientenauflage angeordnet werden. Diese Lösung ist insofern von Nachteil, weil die Phantome zusätzlich verwendet werden, weiterhin zu einem anderen Zeitpunkt verwendet werden und schließlich die sichere und zuverlässige Positionierung der Phantome nicht stets gewährleistet ist

Weiterhin ist bekannt, eine externe Kamera zu verwenden, mittels derer die Position des Patienten im dreidimensionalen Raum exakt vermessen werden kann. Diese Lösung ist insofern von Nachteil, weil die Verwendung der Kamera durch das Bedienpersonal zusätzlich erlernt werden muss und weiterhin auch Behandlungszeit kostet.

Schließlich ist bekannt, mittels eines Lasers eine 3-D-Vermessung des Patienten vorzunehmen. Auch diese Lösung ist zeitaufwendig und umständlich.

Die Aufgabe der vorliegenden Erfindung besteht darin, eine bildgebende medizintechnische Anlage der eingangs genannten Art derart weiterzuentwickeln, dass die Positionierung des Patienten im Rahmen der der Planung zugrunde liegenden Bilderfassung exakt und ohne weiteres erkennbar ist.

Die Aufgabe wird durch eine bildgebende medizintechnische Anlage mit den Merkmalen des Anspruchs 1 gelöst. Vorteilhafte Ausgestaltungen der bildgebenden medizintechnischen Anlage sind Gegenstand der abhängigen Ansprüche 2 bis 11.

Erfindungsgemäß wird eine bildgebende medizintechnische Anlage der eingangs genannten Art dadurch ausgestaltet,
- dass in der Patientenauflage in der Längsrichtung im Wesentlichen über den Verfahrbereich verteilt an ersten Markierungsorten erste Markierungen angeordnet sind,
- dass die ersten Markierungen, sofern sich die jeweilige erste Markierung im Erfassungsbereich befindet, in den generierten Bildern erkennbar sind und
- dass die ersten Markierungsorte und die ersten Markierungen derart aufeinander abgestimmt sind, dass aus den in den generierten Bildern erkennbaren ersten Markierungen der jeweilige erste Markierungsort ableitbar ist.

Die bildgebende medizintechnische Anlage kann nach Bedarf ausgebildet sein. Typischerweise ist die bildgebende medizintechnische Anlage als Magnetresonanzanlage oder als CT-Anlage ausgebildet.

Auf der Patientenauflage wird üblicherweise ein Patient in Längsrichtung angeordnet. Die Richtung von Kopf zu Fuß des Patienten korrespondiert somit (zumindest im Wesentlichen) mit der Längsrichtung des Liegenbretts.

Die Verfahrbarkeit in Längsrichtung stellt eine Minimalanforderung dar. Es ist möglich, dass das Liegenbrett auch in Querrichtung verfahrbar ist. Dies ist jedoch nicht zwingend erforderlich. In der Regel erfolgt das Verfahren des Liegenbretts bei feststehendem Unterbau.

Das Liegenbrett ist, wie bereits erwähnt, zusammen mit der Patientenauflage in der Längsrichtung verfahrbar. Je nach Positionierung des Liegenbretts in der Längsrichtung ist daher nur der eine oder andere Teil der ersten Markierungen in den generierten Bildern erkennbar. Die Bedingung, dass aus den in den generierten Bildern erkennbaren ersten Markierungen der jeweilige erste Markierungsort ableitbar ist, ist nicht in dem Sinne gemeint, dass ermittelt werden kann, dass an einer bestimmten Stelle eine Markierung angeordnet ist. Vielmehr muss aus den erkennbaren ersten Markierungen ermittelbar sein, um welche erste Markierungen es sich handelt. Aufgrund des Umstands, dass bekannt ist, an welchen ersten Markierungsorten sich die jeweilige erkennbare erste Markierung befindet, kann somit auch der Ort dieser ersten Markierungen in den generierten Bildern ermittelt werden.

Hierzu ein Beispiel: Man nehme an, in der Längsrichtung verteilt seien insgesamt 20 erste Markierungen an entsprechenden ersten Markierungsorten angeordnet, nachfolgend als erste Markierung 1 bis 20 bezeichnet, die an den ersten Markierungsorten 1 bis 20 angeordnet sind. In dem generierten Bild seien die Markierungen 3 und 4 erkennbar. Dann reicht es für den Sachverhalt, dass aus den in den generierten Bildern erkennbaren ersten Markierungen der jeweilige erste Markierungsort ableitbar ist, nicht aus, die Markierungen 3 und 4 zu erkennen und demzufolge zu wissen, dass an den zugehörigen Orten erste Markierungen angeordnet sind. Es muss vielmehr möglich sein, aus den in dem generierten Bild erkennbaren Markierungen 3 und 4 abzuleiten, dass es sich um die Markierungen 3 und 4 handelt. Da weiterhin für jede erste Markierung auch der jeweilige erste Markierungsort bekannt ist, ist somit ableitbar, auf welche Orte in dem generierten Bild die ersten Markierungsorte 3 und 4 abgebildet werden. Es ergibt sich also eine Registrierung oder Kalibrierung.

Durch die verbesserte Registrierung oder Kalibrierung ist es möglich, im Rahmen der Bildauswertung und Therapieplanung, die in der Regel von einem Arzt vorgenommen wird, exakt festzulegen, an welchem Ort (bezüglich eines auf die Patientenauflage definierten Koordinatensystems) sich das Organ des Patienten befindet, das später strahlentherapeutisch behandelt werden soll.

Die Ausgestaltung der ersten Markierungen und der ersten Markierungsorte kann nach Bedarf sein.

Beispielsweise ist es möglich, dass die ersten Markierungen zwei kontinuierliche Linien umfassen, die sich in der Längsrichtung gesehen im Wesentlichen über den Verfahrbereich erstrecken, dass die Linien quer zur Längsrichtung gesehen voneinander einen Abstand aufweisen, dass die Abstände der Linien an den Enden des Verfahrbereichs voneinander abweichen und dass der Abstand eine monotone Funktion, insbesondere eine streng monotone Funktion, des Ortes in Längsrichtung ist. Die Linien können insbesondere V-förmig verlaufen. Auch ein treppenartig abgestufter Verlauf ist möglich. Auch Kombinationen hiervon sind möglich.

Alternativ ist es möglich, dass die ersten Markierungen in der Längsrichtung gesehen diskret verteilt sind.

Im Falle einer diskreten Verteilung ergeben sich mehr Freiheitsgrade für die Anordnung der ersten Markierungen. Im einfachsten Fall ist die Anordnung derart, dass ein erster Teil der ersten Markierungen auf einer ersten Verbindungslinie liegt und ein zweiter Teil der ersten Markierungen auf einer zweiten Verbindungslinie liegt und die beiden Verbindungslinien die vorstehend für die kontinuierlichen Linien erläuterten Sachverhalte erfüllen, also beispielsweise monoton und insbesondere streng monoton aufeinander zu oder voneinander weg verlaufen. Es ist aber auch möglich, dass die ersten Markierungen ähnlich einer Binärkodierung (0 = an einer bestimmten Stelle eines Rasters ist eine erste Markierung vorhanden, 1 = an der bestimmten Stelle des Rasters ist keine erste Markierung vorhanden oder eine andersartige erste Markierung vorhanden) mit beispielsweise 4 Bit ausgestaltet sind, so dass die ersten Markierungsorte aus dem "binär kodierten Wert" ermittelt werden können.

Vorzugsweise sind in der Patientenauflage in der Längsrichtung diskret verteilt im Bereich der Außenseiten der Patientenauflage an zweiten Markierungsorten zweite Markierungen angeordnet, die, sofern sich die jeweilige zweite Markierung im Erfassungsbereich befindet, in den generierten Bildern erkennbar sind. Dadurch kann insbesondere eine etwaige Querpositionierung der Patientenauflage genauer erfasst werden.

Die Formulierung "im Bereich der Außenseiten" soll bedeuten, dass die zweiten Markierungen sich zwar in der Nähe der Außenseiten befinden, aber einen Abstand von den Außenseiten aufweisen können. Der Abstand von der jeweiligen Außenseite sollte maximal 15 %, vorzugsweise sogar maximal 10 % der Breite der Patientenauflage betragen.

Die zweiten Markierungsorte können in dem Fall, dass die ersten Markierungen in der Längsrichtung gesehen diskret verteilt sind, auf die Positionierung der ersten Markierungen in der Längsrichtung gesehen abgestimmt sein. Beispielsweise können die zweiten Markierungen in der Längsrichtung gesehen an den gleichen Positionen wie die ersten Markierungen angeordnet sein oder in der Längsrichtung gesehen jeweils in der Mitte ("auf Lücke") zwischen zwei ersten Markierungen angeordnet sein.

Vorzugsweise weist die Patientenauflage in der Längsrichtung verteilt an den Außenseiten vorbereitete Anbaupositionen für Zusatzelemente auf. Dadurch kann die Patientenauflage nach Bedarf mit Zusatzelementen versehen werden. Bei den Zusatzelementen kann es sich beispielsweise um Auflagen für die Arme des Patienten oder um Justierelemente für die exakte Fixierung des Kopfes oder anderer Körperteile des Patienten handeln.

Die Anbaupositionen können, sofern die ersten Markierungen in der Längsrichtung gesehen diskret verteilt sind und/oder die zweiten Markierungen vorhanden sind, in der Längsrichtung gesehen auf die Positionierung der ersten und/oder zweiten Markierungen abgestimmt sein. Beispielsweise können die Anbaupositionen in der Längsrichtung gesehen an den gleichen Positionen wie die ersten und/oder zweiten Markierungen angeordnet sein oder in der Längsrichtung gesehen jeweils in der Mitte ("auf Lücke") zwischen zwei ersten und/oder zweiten Markierungen angeordnet sein.

Vorzugsweise ist die Patientenauflage an ihrer Oberseite flach und ungepolstert ausgebildet. Dadurch ergibt sich bei der Erfassung der Daten mittels des Erfassungssystems eine Liegesituation, die möglichst gut an die spätere Liegesituation im Rahmen der strahlentherapeutischen Maßnahme angeglichen ist. Somit werden die Reproduzierbarkeit bei der Positionierung und die Planbarkeit der strahlentherapeutischen Maßnahme verbessert.

Vorzugsweise weist die Patientenauflage eine mit einer Steuer- und Auswertungseinrichtung der bildgebenden medizintechnischen Anlage datentechnisch verbindbare Schnittstelle auf, über welche eine Kennung über den Typ und/oder die individuelle Patientenauflage an die Steuer- und Auswertungseinrichtung übermittelbar ist. Dadurch kann die Patientenauflage der Steuer- und Auswertungseinrichtung sozusagen selbst mitteilen, um welche Art von Patientenauflage oder gegebenenfalls sogar um welche individuelle Patientenauflage es sich jeweils handelt. Wenn beispielsweise nur der Typ angezeigt wird, ist es möglich, anzuzeigen, dass es sich um eine Patientenauflage für ein Kind oder für einen Patienten einer bestimmten Gewichtsklasse handelt. Wenn die individuelle Patientenauflage angezeigt wird, kann überprüft werden, dass für die Planung der strahlentherapeutischen Maßnahme stets die gleiche Patientenauflage verwendet wird oder für die Planung und die Durchführung der strahlentherapeutischen Maßnahme die gleiche Patientenauflage verwendet wird.

Vorzugsweise weisen die Patientenauflage und das Liegenbrett miteinander zusammenwirkende Positionierelemente auf, so dass die Patientenauflage in der Längsrichtung und orthogonal dazu spielfrei auf dem Liegenbrett angeordnet ist. Diese Ausgestaltung verbessert die Reproduzierbarkeit bei der Positionierung der Patientenauflage auf dem Liegenbrett und damit im Endergebnis des Patienten.

Vorzugsweise sind die Patientenauflage und das Liegenbrett derart aufeinander abgestimmt, dass die Außenseiten der Patientenauflage zumindest abschnittsweise von dem Liegenbrett beabstandet sind. Durch die entsprechenden Lücken können beispielsweise Kabel zu zwischen der Patientenauflage und dem Liegenbrett angeordneten Komponenten geführt werden, beispielsweise im Falle einer Magnetresonanzanlage zu einer Lokalspule.

Vorzugsweise sind die Patientenliege und das Liegenbrett derart aufeinander abgestimmt, dass die Patientenauflage im Bereich zwischen den Außenseiten der Patientenauflage zumindest abschnittsweise von dem Liegenbrett beabstandet ist. Durch diese Abstände können in dem Bereich zwischen der Patientenauflage und dem Liegenbrett Komponenten angeordnet werden, beispielsweise im Falle einer Magnetresonanzanlage eine Lokalspule.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die in Verbindung mit den Zeichnungen näher erläutert werden. Hierbei zeigen in schematischer Darstellung:
- FIG 1: eine bildgebende medizintechnische Anlage von der Seite,
- FIG 2: die bildgebende medizintechnische Anlage von FIG 1 aus einer Richtung II in FIG 1,
- FIG 3: die bildgebende medizintechnische Anlage von FIG 1 in einem modifizierten Betriebszustand,
- FIG 4: eine Patientenauflage,
- FIG 5: ein Bild,
- FIG 6: eine Patientenauflage,
- FIG 7: ein Bild,
- FIG 8: eine Patientenauflage,
- FIG 9: ein Bild und
- FIG 10: ein Liegenbrett und eine Patientenauflage.

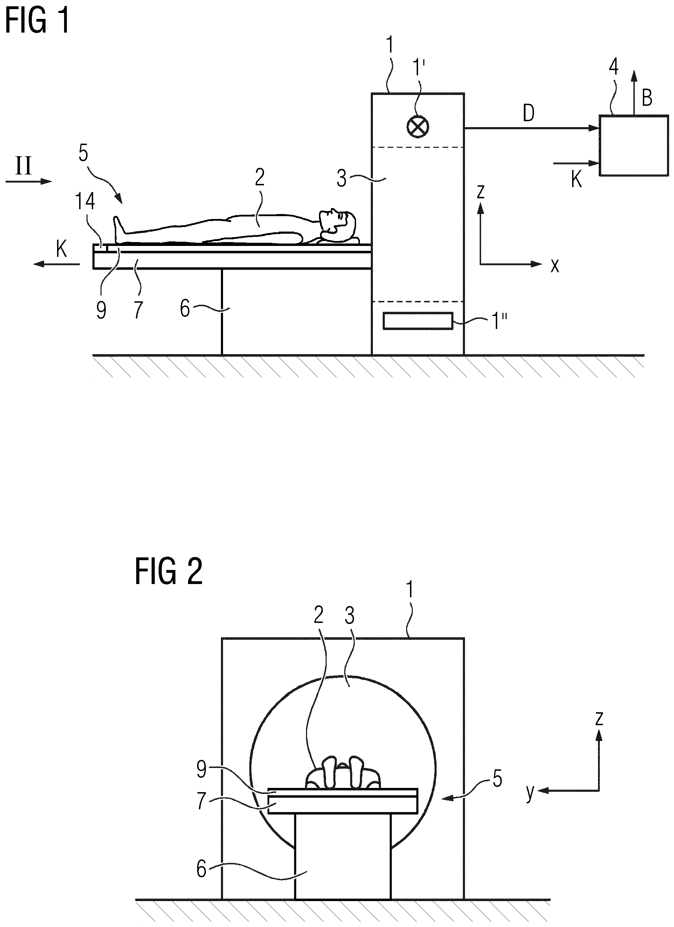

Gemäß den FIG 1 und 2 weist eine bildgebende medizintechnische Anlage ein Erfassungssystem 1 auf. Mittels des Erfassungssystems 1 sind Daten D eines Objekts 2 erfassbar, wenn und soweit sich das Objekt 2 (in der Regel ein Mensch, konkret ein Patient) in einem Erfassungsbereich 3 des Erfassungssystems 1 befindet. Aus den Daten D sind entsprechende Bilder B des Objekts 2 generierbar. Die Bilder B können beispielsweise an eine Person (nicht dargestellt) ausgegeben werden. Die Bilder B können 2D und/oder 3D sein. In dem Ausführungsbeispiel der FIG 1 und 2 ist das Erfassungssystem 1, wie durch eine Röntgenquelle 1' und einen Röntgendetektor 1" schematisch angedeutet ist, als CT-Anlage ausgebildet. Das Erfassungssystem 1 könnte aber auch andersartig ausgebildet sein, beispielsweise als MR-Anlage.

Das Erfassungssystem 1 ist mit einer Steuer- und Auswertungseinrichtung 4 datentechnisch verbunden. Von der Steuer- und Auswertungseinrichtung 4 wird zumindest das Erfassungssystem 1 gesteuert. Weiterhin werden von dem Erfassungssystem 1 dessen Daten D entgegengenommen und wird das Bild B ermittelt. Oftmals werden auch weitere Bestandteile der bildgebenden medizintechnischen Anlage von der Steuer- und Auswertungseinrichtung 4 gesteuert und sind daher datentechnisch mit ihr verbunden.

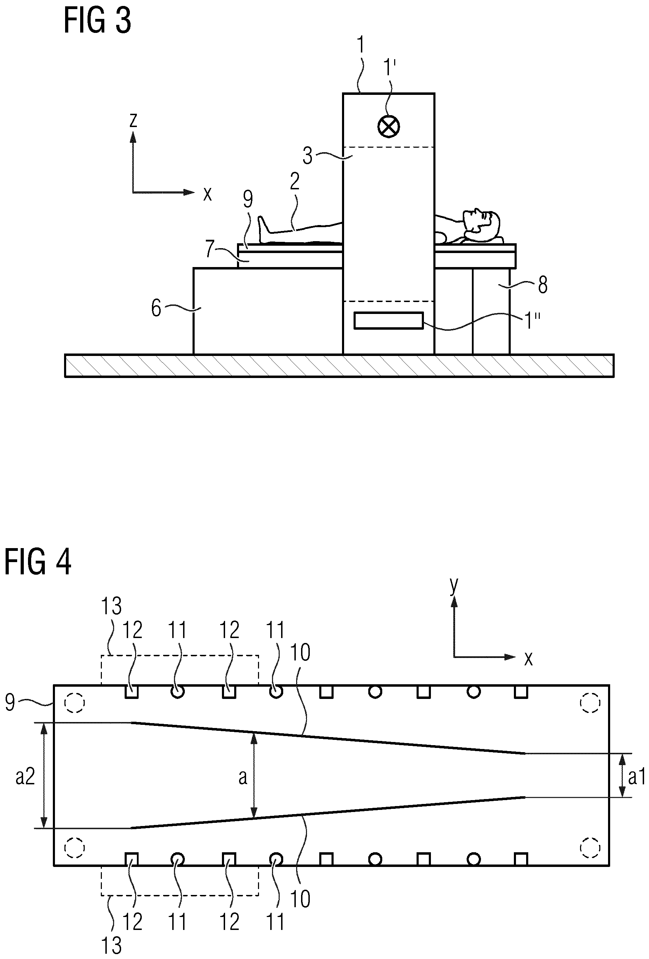

Gemäß den FIG 1 und 2 weist die bildgebende medizintechnische Anlage weiterhin einen Liegentisch 5 auf. Der Liegentisch 5 weist seinerseits einen Unterbau 6 und ein Liegenbrett 7 auf. Es ist möglich, dass der Unterbau 6 fest mit dem Erfassungssystem 1 verbunden ist. Alternativ ist es möglich, dass der Unterbau 6 relativ zum Erfassungssystem 1 beweglich ist oder sogar vom Erfassungssystem 1 lösbar ist. In jedem Fall aber ist das Liegenbrett 7 auf dem Unterbau 6 zumindest in einer Längsrichtung x über einen Verfahrbereich verfahrbar. Beispielsweise zeigt FIG 1 einen Zustand, in dem das Liegenbrett 7 völlig zurückgezogen ist, und zeigt FIG 3 einen Zustand, in dem das Liegenbrett 7 weitgehend ausgefahren ist.

FIG 3 zeigt weiterhin auch eine optionale Ausgestaltung. Im Rahmen dieser Ausgestaltung ist auf der vom Unterbau 6 abgewandten Seite des Erfassungssystems 1 eine Stütze 8 vorhanden. Durch die Stütze 8 können Biegekräfte, die das Liegenbrett 7 im ausgefahrenen Zustand auf den Unterbau 6 ausübt, geringgehalten werden. Die Stütze 8 ist im Rahmen der vorliegenden Erfindung von untergeordneter Bedeutung. Weiterhin könnte - alternativ oder zusätzlich zu der Stütze 8 - auch im Tunnel des Erfassungssystems 1 (= dem Erfassungsbereich 3) eine Führung für das Liegenbrett 7 angeordnet sein.

In manchen Fällen ist das Liegenbrett 7 auch quer zur Längsrichtung x verfahrbar. Die entsprechende Richtung wird nachstehend als Querrichtung y bezeichnet. Die Verfahrbarkeit in Querrichtung y ist im Rahmen der vorliegenden Erfindung von untergeordneter Bedeutung. Auf sie wird daher nachstehend nicht näher eingegangen.

Je nachdem, wie weit das Liegenbrett 7 ausgefahren ist bzw. - hiermit gleichwertig - wie das Liegenbrett 7 in der Längsrichtung x positioniert ist, befinden sich unterschiedliche Bereiche des Liegenbretts 7 in dem Erfassungsbereich 3. Dies gilt zumindest bei feststehendem Unterbau 6, kann aber auch bei einem verfahrbaren Unterbau 6 gelten.

Der Patient (= das Objekt 2) liegt nicht direkt auf dem Liegenbrett 7. Vielmehr ist auf dem Liegenbrett 7 eine Patientenauflage 9 angeordnet, die zusammen mit dem Liegenbrett 7 zumindest in der Längsrichtung x verfahren wird. Im Rahmen der vorliegenden Erfindung ist die Patientenauflage 9 vorzugsweise an ihrer Oberseite flach und ungepolstert ausgebildet. Weiterhin ist die Patientenauflage 9 von oben gesehen üblicherweise rechteckig ausgebildet. Dies ist jedoch von untergeordneter Bedeutung.

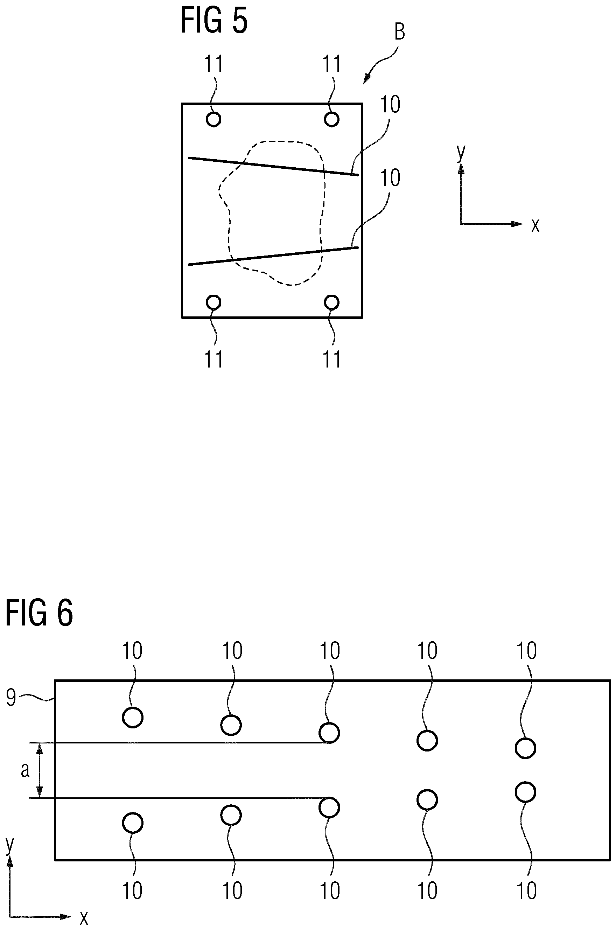

Die Patientenauflage 9 und deren Ausgestaltung stellen wesentliche Aspekte der vorliegenden Erfindung dar. Nachstehend werden in Verbindung mit FIG 4 eine erste mögliche Ausgestaltung der Patientenauflage 9 und in Verbindung mit FIG 5 die sich dadurch ergebende Wirkung in dem generierten Bild B erläutert.

Gemäß FIG 4 sind in der Patientenauflage 9 erste Markierungen 10 angeordnet. Konkret sind die ersten Markierungen 10 in der Längsrichtung x im Wesentlichen über den Verfahrbereich verteilt an ersten Markierungsorten angeordnet. Die ersten Markierungen 10 bestehen aus einem geeigneten Material, so dass die ersten Markierungen 10, sofern sich die jeweilige erste Markierung 10 im Erfassungsbereich 3 befindet, entsprechend der Darstellung in FIG 5 in den generierten Bildern B erkennbar sind. Bei einem als Magnetresonanzanlage ausgebildeten Erfassungssystem 1 können die ersten Markierungen 10 beispielsweise aus einem Material bestehen, das in den Bildern B einem definierten Grauwert erzeugt. Bei einem als CT-Anlage ausgebildeten Erfassungssystem 1 können die ersten Markierungen 10 beispielsweise aus einem Metall bestehen, insbesondere aus Stahl oder Blei. Die ersten Markierungen 10 sind ohne weiteres von einer anatomischen Struktur (in FIG 5 gestrichelt angedeutet) unterscheidbar.

Die ersten Markierungsorte und die ersten Markierungen 10 sind derart aufeinander abgestimmt, dass aus den in den generierten Bildern B erkennbaren ersten Markierungen 10 der jeweilige erste Markierungsort ableitbar ist. Beispielsweise umfassen die ersten Markierungen 10 bei der Ausgestaltung gemäß FIG 4 (mindestens) zwei kontinuierliche Linien. Die Linien erstrecken sich in der Längsrichtung x gesehen im Wesentlichen über den Verfahrbereich. In der Querrichtung y sind die Linien voneinander beabstandet. Der Abstand a der Linien variiert in der Querrichtung y. Es gilt also a = f(x).

Im Falle von zwei Linien könnten die beiden Linien beispielsweise ein X bilden. In diesem Fall könnte aus dem Abstand a der beiden Linien voneinander der Abstand zum Schnittpunkt des X ermittelt werden und aus dem Neigungswinkel das Vorzeichen des Abstands, ob sich also der in dem Bild B enthaltene Abschnitt der beiden Linien in der Längsrichtung x gesehen oberhalb oder unterhalb des Punktes befindet, an dem die beiden Linien sich kreuzen. Ebenso ist es entsprechend der Darstellung in FIG 4 möglich, dass die Abstände der Linien an den Enden des Verfahrbereichs voneinander abweichen, so dass die beiden Linien also an dem einen Ende einen Abstand a1 und am anderen Ende einen Abstand a2 aufweisen, wobei die Abstände a1 und a2 voneinander verschiedene Werte aufweisen. In diesem Fall kann der Abstand a der beiden Linien voneinander von dem einen Ende des Verfahrbereichs zum anderen Ende des Verfahrbereichs beispielsweise eine monotone Funktion des Ortes in Längsrichtung x sein, insbesondere eine streng monotone Funktion. Ein Beispiel eines derartigen funktionalen Verlaufs besteht entsprechend der Darstellung in FIG 4 darin, dass die beiden Linien ein V bilden.

Gemäß FIG 4 sind weiterhin in der Patientenauflage 9 an zweiten Markierungsorten zweite Markierungen 11 angeordnet, in FIG 4 durch kleine Kreise symbolisiert. Die zweiten Markierungen 11 sind in der Längsrichtung x diskret verteilt im Bereich der Außenseiten der Patientenauflage 9 angeordnet. Von den zweiten Markierungen 11 sind in FIG 4 nur einige mit ihrem Bezugszeichen versehen. Auch die zweiten Markierungen 11 können entsprechend der Darstellung in FIG 5 in den generierten Bildern B erkennbar sein, sofern sich die jeweilige zweite Markierung 11 im Erfassungsbereich 3 befindet. Die zweiten Markierungen 11 sind an der Patientenauflage 9 oftmals von einem Menschen direkt erkennbar.

Gemäß FIG 4 weist die Patientenauflage 9 weiterhin in der Längsrichtung x verteilt an den Außenseiten vorbereitete Anbaupositionen 12 auf. Von den Anbaupositionen 12 sind in FIG 4 nur einige mit ihrem Bezugszeichen versehen. Mittels der Anbaupositionen 12 können Zusatzelemente 13 (in FIG 4 gestrichelt angedeutet) an definierten Positionen an der Patientenauflage 9 befestigt werden.

Sowohl das Vorhandensein der zweiten Markierungen 11 als auch das Vorhandensein der Anbaupositionen 12 ist üblich. Beide Sachverhalte sind aber nicht zwingend gegeben. Falls sowohl die zweiten Markierungen 11 als auch die Anbaupositionen 12 vorhanden sind, kann insbesondere die Positionierung der zweiten Markierungen 11 auf die Positionierung der Anbaupositionen 12 abgestimmt sein. Beispielsweise kann in der Längsrichtung x gesehen eine 1:1-Korrespondenz bestehen oder können die Anbaupositionen 12, wie in FIG 4 dargestellt, relativ zu den zweiten Markierungen 11 auf Lücke angeordnet sein.

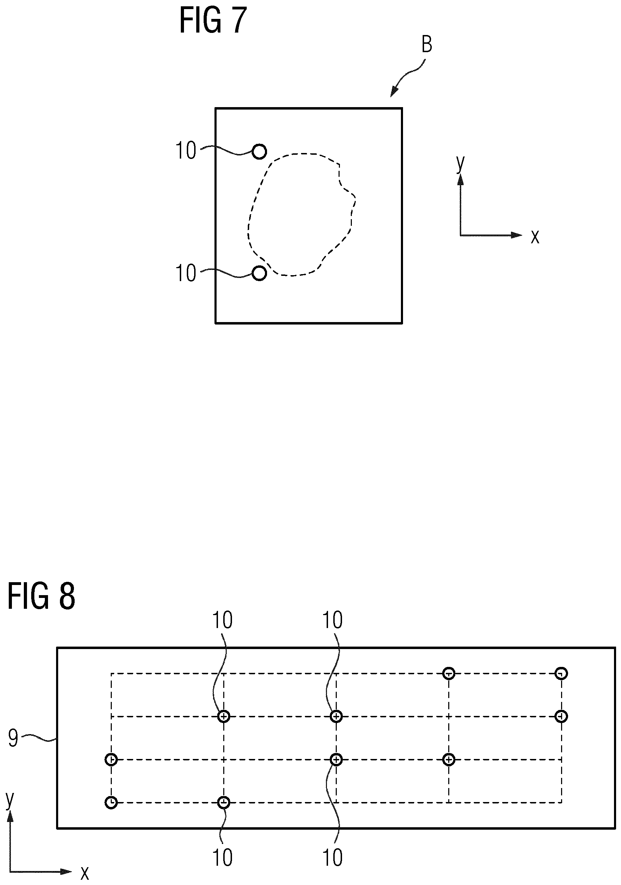

Nachstehend werden in Verbindung mit FIG 6 eine zweite mögliche Ausgestaltung der Patientenauflage 9 und in Verbindung mit FIG 7 die sich dadurch ergebende Wirkung in dem generierten Bild B erläutert.

Gemäß FIG 6 sind - analog zu der Ausgestaltung gemäß FIG 4 - in der Patientenauflage 9 an ersten Markierungsorten erste Markierungen 10 angeordnet. Die ersten Markierungen 10 sind in FIG 6 durch kleine Kreise symbolisiert. Auch die ersten Markierungen 10 der Ausgestaltung von FIG 6 bestehen aus einem geeigneten Material, so dass die ersten Markierungen 10, sofern sich die jeweilige erste Markierung 10 im Erfassungsbereich 3 befindet, entsprechend der Darstellung in FIG 7 in den generierten Bildern B erkennbar sind. Die obigen Ausführungen zu FIG 4 sind in analoger Weise anwendbar.

Im Gegensatz zu der Ausgestaltung von FIG 4 sind bei der Ausgestaltung von FIG 6 die ersten Markierungen 10 in der Längsrichtung x gesehen diskret verteilt. Beispielsweise können entsprechend der Darstellung in FIG 6 die Abstände a der ersten Markierungen 10 in Abhängigkeit vom Ort in der Längsrichtung x gesehen derart variieren, dass jeder Abstand a nur einmal auftritt, so dass der jeweilige Abstand a für den Ort in der Längsrichtung x charakteristisch ist. Falls gewährleistet werden kann, dass in dem generierten Bild B in der Längsrichtung x gesehen an mehreren Orten derartige erste Markierungen 10 erkennbar sind, kann dies gegebenenfalls auch erst durch die gemeinsame Verwertung ermittelbar sein.

Gemäß FIG 6 sind die ersten Markierungen 10 auf zwei Linien angeordnet, die der Querrichtung y voneinander beabstandet sind, wobei der Abstand der Linien in der Querrichtung y streng monoton variiert. Konkret bilden die beiden Linien ein V. Dies ist aber nicht zwingend erforderlich. Insbesondere in dem Fall, dass in dem generierten Bild B in der Längsrichtung x gesehen an mehreren Orten derartige erste Markierungen 10 erkennbar sind, könnten die beiden Linien beispielsweise auch ein X bilden. Gestrichelt ist in FIG 7 wieder eine anatomische Struktur angedeutet.

Auch bei der Ausgestaltung gemäß FIG 6 können die zweiten Markierungen 11 und/oder die Anbaupositionen 12 vorhanden sein. Dies ist in den FIG 6 und 7 lediglich der Übersichtlichkeit halber nicht dargestellt. Die entsprechenden Ausführungen zu FIG 4 sind analog anwendbar. Zusätzlich können die zweiten Markierungen 11 und/oder die Anbaupositionen 12 auch auf die Positionen der ersten Markierungen 10 abgestimmt sein. Beispielsweise kann in der Längsrichtung x gesehen eine 1:1-Korrespondenz bestehen oder kann eine Anordnung auf Lücke gegeben sein.

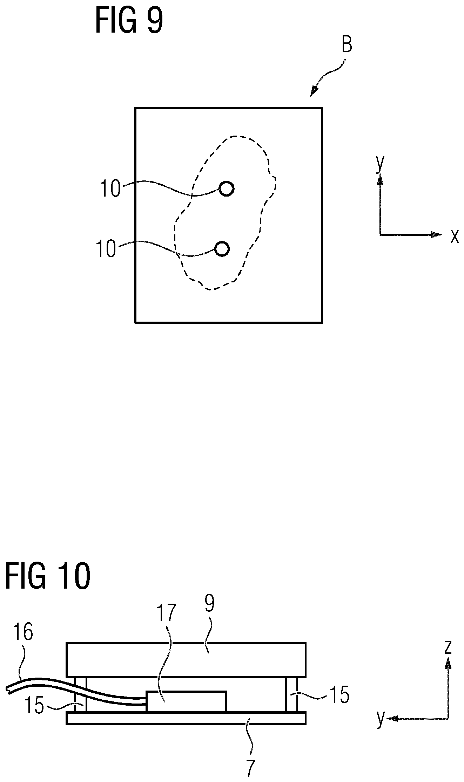

Nachstehend werden in Verbindung mit FIG 8 eine dritte mögliche Ausgestaltung der Patientenauflage 9 und in Verbindung mit FIG 9 die sich dadurch ergebende Wirkung in dem generierten Bild B erläutert.

Gemäß FIG 8 sind - analog zu der Ausgestaltung gemäß FIG 6 - in der Patientenauflage 9 erste Markierungen 10 angeordnet, wobei die ersten Markierungen 10 in der Längsrichtung x gesehen diskret verteilt sind. Von den ersten Markierungen 10 sind in FIG 8 nur einige mit ihrem Bezugszeichen versehen. Wie bei der Ausgestaltung gemäß FIG 6 auch bestehen die ersten Markierungen 10 der Ausgestaltung von FIG 8 aus einem geeigneten Material, so dass die ersten Markierungen 10, sofern sich die jeweilige erste Markierung 10 im Erfassungsbereich 3 befindet, entsprechend der Darstellung in FIG 9 in den generierten Bildern B erkennbar sind. Die obigen Ausführungen zu FIG 4 und zu FIG 6 sind in analoger Weise anwendbar. Gestrichelt ist in FIG 9 wieder eine anatomische Struktur angedeutet.

Bei der Ausgestaltung gemäß FIG 8 existiert ein Raster, in dem die ersten Markierungen 10 angeordnet sein können. Das Raster ist in FIG 8 durch gestrichelte Linien angedeutet. Die Orte, an denen die ersten Markierungen 10 angeordnet sein können, werden nachstehend als mögliche Markierungsorte bezeichnet. Durch Auswertung des Sachverhalts, an welchen der möglichen Markierungsorte tatsächlich eine erste Markierung 10 angeordnet ist, kann bei geeigneter "Kodierung" daher der Ort in der Längsrichtung x in dem generierten Bild B ermittelt werden.

FIG 8 zeigt rein beispielhaft eine 2-aus-4-Kodierung, bei der insgesamt sechs verschiedene Kodierungen möglich sind und vorliegend fünf dieser Kodierungen verwendet werden. Es sind aber auch andere Kodierungen möglich.

Auch bei der Ausgestaltung gemäß FIG 8 können die zweiten Markierungen 11 und/oder die Anbaupositionen 12 vorhanden sein. Dies ist in den FIG 8 und 9 lediglich der Übersichtlichkeit halber nicht dargestellt. Die entsprechenden Ausführungen zu FIG 6 sind analog anwendbar.

Wie bereits erwähnt, wird zumindest das Erfassungssystem 1 von der Steuer- und Auswertungseinrichtung 4 gesteuert. Vorzugsweise weist die Patientenauflage 9 entsprechend der Darstellung in FIG 1 eine Schnittstelle 14 auf, die mit der Steuer-und Auswertungseinrichtung 4 datentechnisch verbindbar ist. Die Schnittstelle 14 kann beispielsweise als RFID-Transponder ausgebildet sein. Auch andere Ausgestaltungen sind möglich. In der Schnittstelle 14 kann eine Kennung K hinterlegt sein. Die Kennung K kann an die Steuer- und Auswertungseinrichtung 4 übermittelt werden. Die Kennung K kann beispielsweise charakteristisch für den Typ der Patientenauflage 9 und/oder die individuelle Patientenauflage 9 sein.

Vorzugsweise weisen weiterhin die Patientenauflage 9 und das Liegenbrett 7 miteinander zusammenwirkende Positionierelemente 15 auf. Die Positionierelemente 15 können beispielsweise gemäß FIG 10 - zusätzlich in FIG 4 und auch dort nur gestrichelt angedeutet - auf Seiten der Patientenliege 9 fußartige Vorsprünge und auf Seiten des Liegenbretts 7 korrespondierende Ausnehmungen aufweisen. In diesem Fall können die Vorsprünge in die Ausnehmungen eintauchen und dadurch die Patientenauflage 9 relativ zum Liegenbrett 7 spielfrei fixieren. Die Fixierung erfolgt in aller Regel zumindest in der Längsrichtung x und in der Querrichtung y, oftmals zusätzlich auch in einer sowohl zur Längsrichtung x als auch zur Querrichtung y orthogonalen Höhenrichtung z.

Vorzugsweise sind weiterhin entsprechend der Darstellung in FIG 10 die Patientenauflage 9 und das Liegenbrett 7 derart aufeinander abgestimmt, dass die Außenseiten der Patientenauflage 9 zumindest abschnittsweise von dem Liegenbrett 7 beabstandet sind. Dadurch können beispielsweise in dem entsprechenden Bereich Kabel 16 geführt werden.

Schließlich sind entsprechend der Darstellung in FIG 10 die Patientenauflage 9 und das Liegenbrett 7 vorzugsweise derart aufeinander abgestimmt, dass die Patientenauflage 9 im Bereich zwischen den Außenseiten der Patientenauflage 9 zumindest abschnittsweise von dem Liegenbrett 7 beabstandet ist. Dadurch können in dem entsprechenden Bereich Nutzkomponenten 17 angeordnet werden, beispielsweise im Falle einer Magnetresonanzanlage eine Lokalspule.

### Zusammengefasst betrifft die vorliegende Erfindung somit folgenden Sachverhalt

Eine bildgebende medizintechnische Anlage weist ein Erfassungssystem 1 auf, mittels dessen Daten D eines in einem Erfassungsbereich 3 des Erfassungssystems 1 befindlichen Objekts 2 erfassbar sind, aus denen entsprechende Bilder B des Objekts 2 generierbar sind. Die bildgebende medizintechnische Anlage weist weiterhin einen Liegentisch 5 auf, der einen Unterbau 6 und ein Liegenbrett 7 aufweist. Das Liegenbrett 7 ist auf dem Unterbau 6 zumindest in einer Längsrichtung x über einen Verfahrbereich verfahrbar, so dass sich je nach Positionierung des Liegenbretts 7 in der Längsrichtung x unterschiedliche Bereiche des Liegenbretts 7 in dem Erfassungsbereich 3 befinden. Auf dem Liegenbrett 7 ist eine Patientenauflage 9 angeordnet, die zusammen mit dem Liegenbrett 7 zumindest in der Längsrichtung x verfahren wird. In der Patientenauflage 9 sind in der Längsrichtung x im Wesentlichen über den Verfahrbereich verteilt an ersten Markierungsorten erste Markierungen 10 angeordnet. Die ersten Markierungen 10 sind, sofern sich die jeweilige erste Markierung 10 im Erfassungsbereich 3 befindet, in den generierten Bildern B erkennbar. Die ersten Markierungsorte und die ersten Markierungen 10 sind derart aufeinander abgestimmt, dass aus den in den generierten Bildern B erkennbaren ersten Markierungen 10 der jeweilige erste Markierungsort ableitbar ist.

Die vorliegende Erfindung weist viele Vorteile auf. Aufgrund der ersten Markierungen 10 ist eine sehr genaue Ermittlung des Ortes in der Längsrichtung x (und manchmal auch in der Querrichtung y) möglich. Die Genauigkeit liegt im Bereich von 2 mm oder besser, manchmal sogar im Bereich von 1 mm. Die ersten Markierungen 10 und gegebenenfalls auch die zweiten Markierungen 11 können oftmals mittels einer automatisierten Bildanalyse ermittelt werden. Lagerungen des Objekts 2 (= des Patienten) sind sehr gut reproduzierbar oder zumindest vergleichbar. Die Patientenauflage kann auch zur Qualitätssicherung beim Verschieben des Liegenbretts 7 in der Längsrichtung x verwendet werden.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Bildgebende medizintechnische Anlage,
- wobei die bildgebende medizintechnische Anlage ein Erfassungssystem (1) aufweist, mittels dessen Daten (D) eines in einem Erfassungsbereich (3) des Erfassungssystems (1) befindlichen Objekts (2) erfassbar sind, aus denen entsprechende Bilder (B) des Objekts (2) generierbar sind,
- wobei die bildgebende medizintechnische Anlage einen Liegentisch (5) aufweist,
- wobei der Liegentisch (5) einen Unterbau (6) und ein Liegenbrett (7) aufweist,
- wobei das Liegenbrett (7) auf dem Unterbau (6) zumindest in einer Längsrichtung (x) über einen Verfahrbereich verfahrbar ist, so dass sich je nach Positionierung des Liegenbretts (7) in der Längsrichtung (x) unterschiedliche Bereiche des Liegenbretts (7) in dem Erfassungsbereich (3) befinden,
- wobei auf dem Liegenbrett (7) eine Patientenauflage (9) angeordnet ist, die zusammen mit dem Liegenbrett (7) zumindest in der Längsrichtung (x) verfahren wird,
**dadurch gekennzeichnet ,**
- **dass** in der Patientenauflage (9) in der Längsrichtung (x) im Wesentlichen über den Verfahrbereich verteilt an ersten Markierungsorten erste Markierungen (10) angeordnet sind,
- **dass** die ersten Markierungen (10), sofern sich die jeweilige erste Markierung (10) im Erfassungsbereich (3) befindet, in den generierten Bildern (B) erkennbar sind und
- **dass** die ersten Markierungsorte und die ersten Markierungen (10) derart aufeinander abgestimmt sind, dass aus den in den generierten Bildern (B) erkennbaren ersten Markierungen (10) der jeweilige erste Markierungsort ableitbar ist.

2. Anlage nach Anspruch 1,
**dadurch gekennzeichnet ,**
**dass** die ersten Markierungen (10) zwei kontinuierliche Linien umfassen, die sich im Wesentlichen über den Verfahrbereich erstrecken, dass die Linien quer zur Längsrichtung (x) gesehen voneinander einen Abstand (a) aufweisen und dass der Abstand (a) der Linien quer zur Längsrichtung (x) gesehen variiert.

3. Anlage nach Anspruch 2,
**dadurch gekennzeichnet ,**
**dass** die Abstände (a) der Linien an den Enden des Verfahrbereichs voneinander abweichen und dass der Abstand (a) eine monotone Funktion, insbesondere eine streng monotone Funktion, des Ortes in Längsrichtung (x) ist.

4. Anlage nach Anspruch 1,
**dadurch gekennzeichnet ,**
**dass** die ersten Markierungsorte in der Längsrichtung (x) gesehen diskret verteilt sind.

5. Anlage nach einem der obigen Ansprüche,
**dadurch gekennzeichnet ,**
**dass** in der Patientenauflage (9) in der Längsrichtung (x) diskret verteilt im Bereich der Außenseiten der Patientenauflage (9) an zweiten Markierungsorten zweite Markierungen (11) angeordnet sind, die, sofern sich die jeweilige zweite Markierung (11) im Erfassungsbereich (3) befindet, in den generierten Bildern (B) erkennbar sind.

6. Anlage nach einem der obigen Ansprüche,
**dadurch gekennzeichnet ,**
**dass** die Patientenauflage (9) in der Längsrichtung (x) verteilt an den Außenseiten vorbereitete Anbaupositionen (12) für Zusatzelemente (13) aufweist.

7. Anlage nach einem der obigen Ansprüche,
**dadurch gekennzeichnet ,**
**dass** die Patientenauflage (9) an ihrer Oberseite flach und ungepolstert ausgebildet ist.

8. Anlage nach einem der obigen Ansprüche,
**dadurch gekennzeichnet ,**
**dass** die Patientenauflage (9) eine mit einer Steuer- und Auswertungseinrichtung (4) der bildgebenden medizintechnischen Anlage datentechnisch verbindbare Schnittstelle (14) aufweist, über welche eine Kennung (K) über den Typ und/oder die individuelle Patientenauflage (9) an die Steuer- und Auswertungseinrichtung (4) übermittelbar ist.

9. Anlage nach einem der obigen Ansprüche,
**dadurch gekennzeichnet ,**
**dass** die Patientenauflage (9) und das Liegenbrett (7) miteinander zusammenwirkende Positionierelemente (15) aufweisen, so dass die Patientenauflage (9) in der Längsrichtung (x) und orthogonal dazu spielfrei auf dem Liegenbrett (7) angeordnet ist.

10. Anlage nach einem der obigen Ansprüche,
**dadurch gekennzeichnet ,**
**dass** die Patientenauflage (9) und das Liegenbrett (7) derart aufeinander abgestimmt sind, dass die Außenseiten der Patientenauflage (9) zumindest abschnittsweise von dem Liegenbrett (7) beabstandet sind.

11. Anlage nach einem der obigen Ansprüche,
**dadurch gekennzeichnet ,**
**dass** die Patientenliege (9) und das Liegenbrett (7) derart aufeinander abgestimmt sind, dass die Patientenauflage (9) im Bereich zwischen den Außenseiten der Patientenauflage (9) zumindest abschnittsweise von dem Liegenbrett (7) beabstandet ist.
